Europäisches Patentamt

European Patent Office

Office européen des brevets

(11) Veröffentlichungsnummer: **0 331 881**
**A1**

## (12) EUROPÄISCHE PATENTANMELDUNG

(21) Anmeldenummer: 89100759.3

(22) Anmeldetag: 18.01.89

(51) Int. Cl.⁴: **F16M 11/22 , A61N 1/16**

(30) Priorität: 05.03.88 DE 8803014 U

(43) Veröffentlichungstag der Anmeldung:
**13.09.89 Patentblatt 89/37**

(84) Benannte Vertragsstaaten:
**AT BE CH DE ES FR GB GR IT LI LU NL SE**

(71) Anmelder: **Oehme, Rainer**
**Moorstrasse 10**
**D-3033 Schwarmstedt(DE)**

(72) Erfinder: **Oehme, Rainer**
**Moorstrasse 10**
**D-3033 Schwarmstedt(DE)**

(74) Vertreter: **Schlagwein, Udo, Dipl.-Ing.**
**Anwaltsbüro Ruppert & Schlagwein**
**Frankfurter Strasse 34**
**D-6350 Bad Nauheim(DE)**

(54) **Vorrichtung mit einem eine zentrale Stativsäule aufweisenden Stativ.**

(57) Eine zentrale Stativsäule (3) ist über einem pyramidenförmigen Körper angeordnet. Die Stativsäule (3) hat Beine (4, 5), welche gegen die Basis des pyramidenförmigen Körpers (1) anliegen. Dadurch zentrieren die Beine (4, 5) die Stativsäule (3) auf dem pyramidenförmigen Körper (1).

Fig. 1

EP 0 331 881 A1

Vorrichtung mit einem eine zentrale Stativsäule aufweisenden Stativ

Die Neuerung betrifft ein Vorrichtung mit einem eine zentrale Stativsäule aufweisenden Stativ und einem pyramidenförmigen Körper, über welchen das Stativ mit mindestens zwei Beinen greift.

Vorrichtungen dieser Art sind allgemein bekannt und gebräuchlich. Sie werden beispielsweise im therapeutischen Bereich zum Halten von Elektroden unterschiedlichster Art eingesetzt. Dabei ist es erforderlich, daß die Stativsäule genau fluchtend zur Hochachse des pyramidenförmigen Körpers ausgerichtet wird, was eine gewisse Mühe macht und häufig nicht mit der erforderlichen Sorgfalt geschieht.

Der Neuerung liegt die Aufgabe zugrunde, eine Vorrichtung der eingangs genannten Art derart zu gestalten, daß das Ausrichten der Stativachse nach der Hochachse des pyramidenförmigen Körpers möglichst einfach und exakt erfolgen kann.

Diese Aufgabe wird neuerungsgemäß dadurch gelöst, daß die Beine gegen die Basis des pyramidenförmigen Körpers anliegen.

Durch diese Gestaltung läßt sich das Stativ zwangsläufig nur so über den pyramidenförmigen Körper stellen, daß seine Stativsäule zumindest in einer Koordinatenrichtung mit der Hochachse des pyramidenförmigen Körpers fluchtet. Dadurch wird das Ausrichten des Statives wesentlich erleichtert.

Das Stativ ist zwangsläufig in beiden Koordinatenrichtungen ausgerichtet und fluchtet notwendigerweise mit der Hochachse des dazugehörigen, pyramidenförmigen Körpers, sobald es von oben her über den pyramidenförmigen Körper gesetzt ist, wenn gemäß einer vorteilhaften Ausgestaltung der Neuerung das Stativ insgesamt vier Beine hat, welche zwischen den Ecken gegen die Basis der Pyramide an ihren Bodenkanten anliegen.

Eine konstruktiv besonders einfache Ausführungsform der Neuerung besteht darin, daß die Beine entlang der zur Pyramidenspitze verlaufenden Kanten geführt sind und jeweils mit einem Eckstück aufstehen, welches an jeder bodenseitigen Ecke über den Eckbereich zweier bodenseitiger Pyramidenkanten greift.

Das Stativ dient nicht nur als Halterung für Elektroden oder sonstige Bauteile, sondern zugleich als eine in seiner Gesamtheit Energie aufnehmende Antenne, wenn gemäß einer anderen Ausgestaltung der Erfindung die Stativsäule und ihre Beine aus einem elektrisch leitenden Material bestehen und eine Antenne bilden.

Das Stativ kann gleichzeitig zum Zusammenhalten der vier den pyramidenförmigen Körper bildenden Dreiecksflächen dienen, wenn sich die jeweils untere Kante jeder Dreiecksfläche gegen die Beine des Stativs abstützt.

Die neuerungsgemäße Vorrichtung kann zum Halten beispielsweise mehrerer Elektroden dienen, wenn die Stativsäule am oberen Ende zumindest zwei horizontal sich erstreckende Ausleger zum Tragen von jeweils einer Elektrode hat.

Am Stativ können insgesamt beispielsweise vier Elektroden befestigt werden und eine Ausrichtung der Elektrodenanordnung insgesamt in eine gewünschte Winkelstellung ist möglich, wenn gemäß einer anderen Ausgestaltung der Neuerung insgesamt vier Ausleger kreuzförmig auf einer drehbar auf der Stativsäule angeordneten Aufnahme vorgesehen sind.

Die Neuerung läßt zahlreiche Ausführungsformen zu. Zur weiteren Verdeutlichung ihres Grundprinzips sind mehrere davon in der Zeichnung dargestellt und werden nachfolgend beschrieben. Diese zeigt in

Fig. 1 eine Seitenansicht der neuerungsgemäßen Vorrichtung,

Fig. 2 eine Draufsicht auf die Vorrichtung nach Figur 1,

Fig. 3 einen horizontalen Schnitt durch eine weitere Ausführungsform der Vorrichtung im Bereich des unteren Endes ihrer Stativsäule,

Fig. 4 eine Seitenansicht des unteren Bereiches einer weiteren Ausführungsform der Vorrichtung nach der Neuerung.

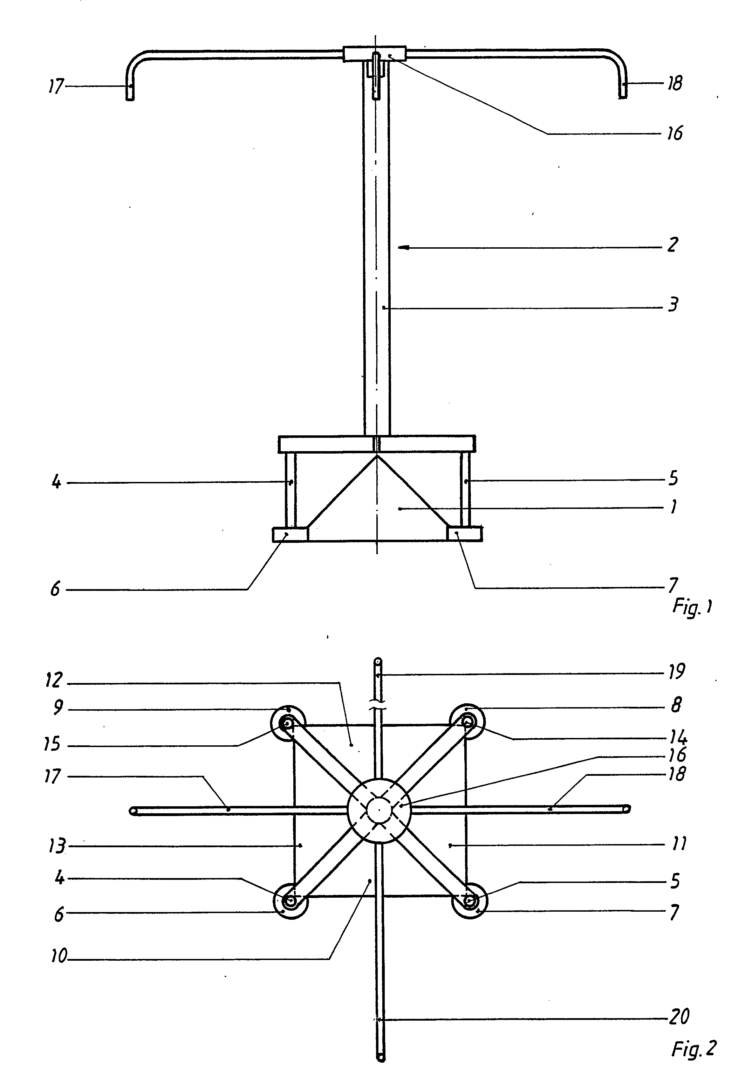

Die Figur 1 zeigt einen pyramidenförmigen Körper 1, über dem ein Stativ 2 angeordnet ist. Dieses Stativ 2 hat eine zentrale Stativsäule 3, welche auf vier Beinen steht, von denen zwei Beine 4, 5 in Figur 1 zu sehen sind. Die Beine 4, 5 stützen sich auf jeweils einem Eckstück 6, 7, 8, 9 ab. Diese Eckstücke 6, 7, 8, 9 umgreifen jeweils eine Ecke der Basis des pyramidenförmigen Körpers 1. Der pyramidenförmige Körper 1 kann aus vier nicht miteinander verbundenen, lediglich aneinanderliegenden Dreiecksflächen 10, 11, 12, 13 bestehen, die durch die Eckstücke 6 - 9 daran gehindert werden, nach außen wegzurutschen.

Die Figur 1 zeigt weiterhin, daß die Stativsäule 3 am oberen Ende eine drehbare Aufnahme 16 hat, in der Ausleger 17, 18 gehalten sind. Diese können beispielsweise Elektroden tragen oder selbst als Elektrode ausgebildet sein.

Die Figur 2 läßt erkennen, daß die Beine 4, 5 und die in dieser Figur zusätzlich zu erkennenden Beine 14, 15 entlang der Kanten des pyramidenförmigen Körpers 1 geführt sind. Ebenso zeigt diese Figur, daß die Aufnahme 16 insgesamt vier Ausleger 17, 18, 19, 20 haltert, welche kreuzförmig angeordnet sind.

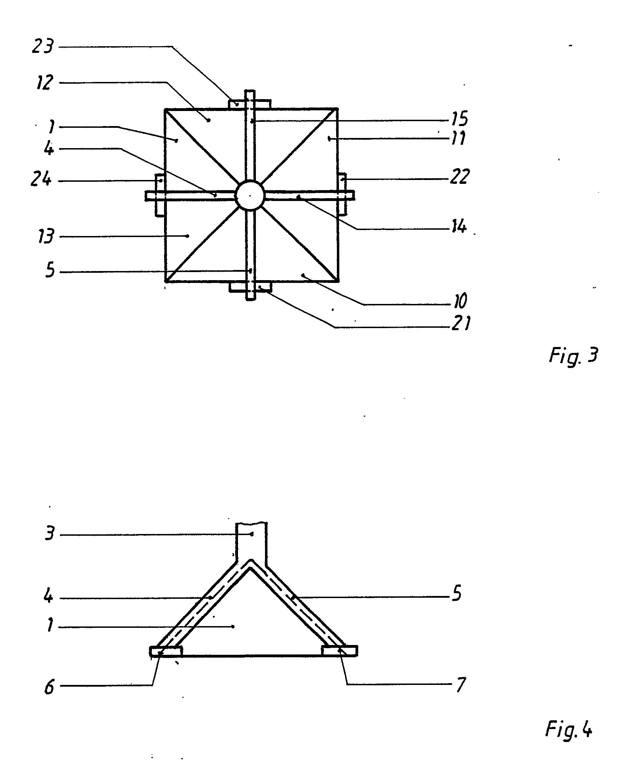

Bei der Ausführungsform nach Figur 3 sind die Beine 4, 5, 14, 15 nicht zu den Ecken des pyramidenförmigen Körpers 1 geführt, sondern verlaufen genau zwischen den Ecken. Statt der Eckstücke liegen bei dieser Ausführungsform an den Beinen 4, 5, 14, 15 angebrachte Halteklötzchen 21, 22, 23, 24 gegen den pyramidenförmigen Körper 1 an und halten seine separaten Dreiecksflächen 10 - 13 zusammen.

Die Figur 4 zeigt am Beispiel der Beine 4, 5, daß diese auch jeweils genau parallel der Kanten des pyramidenförmigen Körpers 1 verlaufen können.

**Ansprüche**

1. Vorrichtung mit einem eine zentrale Stativsäule aufweisenden Stativ und einem pyramidenförmigen Körper, über welchen das Stativ mit mindestens zwei Beinen greift, dadurch gekennzeichnet, daß die Beine (4, 5, 14, 15) gegen die Basis des pyramidenförmigen Körpers (1) anliegen.

2. Vorrichtung nach Anspruch 1, dadurch gekennzeichnet, daß sie insgesamt vier Beine (4, 5, 14, 15) hat, welche zwischen den Ecken gegen die Basis des pyramidenförmigen Körpers (1) an ihren Bodenkanten anliegen.

3. Vorrichtung nach Anspruch 1, dadurch gekennzeichnet, daß die Beine (4, 5, 14, 15) entlang der zur Spitze des pyramidenförmigen Körpers (1) verlaufenden Kanten geführt sind und jeweils mit einem Eckstück (6 - 9) aufstehen, welches an jeder bodenseitigen Ecke über den Eckbereich zweier bodenseitiger Pyramidenkanten greift.

4. Vorrichtung nach zumindest einem der vorangehenden Ansprüche, dadurch gekennzeichnet, daß die Stativsäule (3) und ihre Beine (4, 5, 14, 15) aus einem elektrisch leitenden Material bestehen und eine Antenne bilden.

5. Vorrichtung nach zumindest einem der vorangehenden Ansprüche, dadurch gekennzeichnet, daß der pyramidenförmige Körper (1) aus vier aneinanderliegenden, nicht miteinander verbundenen Dreiecksflächen (10 - 13) besteht, welche sich mit ihrer jeweils unteren Kante gegen die Beine (4, 5, 14, 15) des Stativs (2) abstützen.

6. Vorrichtung nach zumindest einem der vorangehenden Ansprüche, dadurch gekennzeichnet, daß die Stativsäule (3) am oberen Ende zumindest zwei horizontal sich erstreckende Ausleger zum Tragen von jeweils einer Elektrode hat.

7. Vorrichtung nach zumindest einem der vorangehenden Ansprüche, dadurch gekennzeichnet, daß insgesamt vier Ausleger kreuzförmig in einer drehbar auf der Stativsäule angeordneten Aufnahme vorgesehen sind.

Fig. 1

Fig. 2

Fig. 3

Fig. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-3 630 523 (OEHME)<br>* Spalte 2, Zeile 67 - Spalte 3, Zeile 9; Figur 2 *<br>--- | 1,3 | F 16 M 11/22<br>A 61 N 1/16 |
| Y | US-A-2 069 590 (NEUMANN)<br>* Seite 6, linke Spalte, Zeile 53 - rechte Spalte, Zeile 38; Figur 6 *<br>--- | 1,3 | |
| A | FR-A-1 057 531 (SANCHEZ)<br>* Insgesamt *<br>--- | 1,4,6;7 | |
| A | DE-A-3 418 426 (KRÜHLER)<br>* Seite 8, Zeilen 24-31; Seite 9, Zeilen 6-9; Figuren 1,2 *<br>--- | 4,6 | |
| A | DE-A-3 543 765 (KARREMANN)<br>* Seite 8, Zeile 65 - Seite 9, Zeile 17;. Figuren 2,4 *<br>--- | 5 | |
| P,X | DE-U-8 803 014 (OEHME)<br>* Insgesamt *<br>--- | 1-7 | |
| A | DE-U-8 711 136 (OEHME)<br>--- | | RECHERCHIERTE SACHGEBIETE (Int. Cl.4) |
| A | FR-A-1 531 886 (DUMAS)<br>--- | | F 16 M<br>A 61 N |
| A | GB-A- 125 572 (WIGHT)<br>----- | | |

Der vorliegende Recherchenbericht wurde für alle Patentansprüche erstellt

| Recherchenort | Abschlußdatum der Recherche | Prüfer |
|---|---|---|
| DEN HAAG | 12-06-1989 | BARON C. |